# FASCICULE DE BREVET EUROPEEN

(11) **EP 1 584 336 B1**
(45) Date de publication et mention de la délivrance du brevet: **13.08.2008**
(21) Numéro de dépôt: 04290559.6
(22) Date de dépôt: 02.03.2004
(51) Int. Cl.: A61L 9/01, A61L 9/04, A61L 9/12

(54) **Procédé et dispositif d'odorisation et de désodorisation d'un gaz avec un support olfactif de type alumine**
Verfahren und Vorrichtung zur Odorierung und Desodorierung von Gasen mit Aluminiumoxid als Geruchsträger
Method and device for gas odorisation and deodorisation with an alumina-based olfactory support

(43) Date de publication de la demande: 12.10.2005
(73) Titulaire: Cool Sarl., 07800 La Voulte sur Rhône (FR)
(72) Inventeur: Berrebi, David, 07800 La Voulte sur Rhône (FR)
(74) Mandataire: Andreeff, François

(56) Documents cités:
- EP-A- 1 285 669
- FR-A- 2 816 521
- US-A- 5 573 984
- US-A- 5 637 401
- DATABASE WPI Section Ch, Week 198218 Derwent Publications Ltd., London, GB; Class A97, AN 1982-36331E XP002246266 & JP 57 052458 A (DUSKIN FRANCHISE KK) 27 mars 1982 (1982-03-27)

## Description

La demanderesse développe des produits dans le domaine de la parfumerie à base d'huiles essentielles et de synthèse, des supports olfactifs, du traitement de gaz et notamment de l'air (air ambiant par exemple), et plus généralement des produits qui font appel au sens olfactif.

Dans les lieux publics ou privés, pour le compte de sociétés ou d'applications industrielles.

Le principe fondamental réside dans l'utilisation d'alumine activée comme support olfactif de base. En effet, l'alumine activée, lorsqu'elle est imprégnée d'une solution odorante d'huiles essentielles et/ou de synthèses et soumise ou non à l'effet d'un fluide chaud ou froid, agit comme un support olfactif judicieux et performant en vue de diffuser dans l'air ambiant les arômes de la dite solution, considérant les impératifs de persistance olfactive.

L'objet de la présente invention est un perfectionnement important d'un principe décrit dans le brevet européen de la demanderesse EP-A 01 402 461 6 du 26 septembre 2001 (priorité française du 21 Août 2001). Ce brevet concerne la dépollution d'air et l'odorification d'air au moyen d'un support renfermant un agent de dépollution ou un agent d'odorification.

On peut dire que l'alumine activée est un support olfactif intelligent en ce sens qu'il peut s'adapter à différents modes de diffusion d'odeurs et permettre une évaporation contrôlée de la solution odorante imprégnée. Dans le cadre de la présente invention, l'alumine activée a un caractère bi-modal ainsi qu'il sera expliqué ci-dessous.

Plusieurs types d'alumines activées sont nécessaires pour répondre à diverses applications.
Les alumines concernées sont, par exemple, l'alumine activée 2-5 grade A, l'alumine activée 2,5-5 grade D, l'alumine activée DR8-13, l'alumine activée 3-6 EXT, l'alumine activée 4-8 grade D, l'alumine activée 4-8 grade A, l'alumine activée CR/SU 4-8, Alumine ALCOA F200 1/8", Alumine Sphéralite 513, Alumine ALCOA DD431-3/16. Cette liste d'alumines commerciales n'est pas limitative.

L'alumine activée est un support qui se présente sous la forme de perles ou d'extrudés ou tout autre forme, micro et macro poreuse, sur lequel on imprègne tout ou partie de son volume poreux par au moins une huile essentielle ou au moins une solution odorante d'huiles essentielles et/ou de synthèses.

La solution odorante qui sert à l'imprégnation peut contenir des actifs bactéricides, fongicides, et/ou anti-acariens, par l'adjonction de pyréthrine ou composé équivalent, de phéromones connues à ce jour, et/ou d'huiles essentielles dans la formulation de la solution.

Le support d'alumine activée est d'abord imprégné d'une solution odorante. Puis, conditionné ou pas, il est soumis ou non à l'effet d'un fluide chaud ou froid, le fluide étant le gaz ou l'air, c'est là véritablement qu'il libère les arômes de la dite solution ; c'est en cela que l'on peut considérer que le support d'alumine activée est un support olfactif et qu'il est idéal pour tous les dispositifs d'odorification, de désodorisation, et de purification d'un gaz ou de l'air, air ambiant par exemple.

Le support d'alumine activée peut être associé à un ou plusieurs autres supports, de types minéraux ou végétaux, imprégnés eux aussi d'une solution odorante, en vue de servir une fonction d'odorisation particulière.

Le support d'alumine activée imprégné peut être conditionné dans des cassettes ou des recharges spécifiques ou être accueilli simplement en vrac dans le réceptacle d'un dispositif de type diffuseur d'arômes.

L'objet de la présente invention consiste à placer dans le gaz ou dans l'air qui circule, des dispositifs adéquats qui contiennent des supports olfactifs de type alumine activée imprégnée, associés ou non à d'autres supports odorants et dont la fonction et de donner ou redonner une odeur à l'air ambiant.

L'invention consiste donc à faire circuler le dit gaz (l'air notamment), chaud ou froid, à travers une enceinte renfermant un support contenant de l'alumine activée de préférence imprégnée d'une solution odorante à base d'au moins une huile essentielle et/ou d'au moins une huile de synthèse.

La description qui suit concerne l'air et l'alumine activée.

### I. Pour l'odorisation et la désodorisation:

Il s'agit d'obtenir un support micro ou macro poreux du type alumine activée sur lequel on imprègne tout ou partie de son volume poreux par au moins une huile essentielle ou au moins une solution odorante d'huiles essentielles et/ou de synthèses, dit le support olfactif.

Le volume poreux est de préférence compris entre 20 et 70 cm3 pour 100 grammes d'alumine activée, avec de préférence une surface spécifique allant de 200 à 500 m2/grammes (et plus particulièrement 200 à 300 m2/grammes) ; on peut occuper le volume poreux de façon à ce que les huiles essentielles de la solution imprégnée se diffusent lentement.

Afin de doser et/ou équilibrer les quantités d'huiles essentielles émises dans l'air à odoriser, on agit sur la rhéologie du système support/huile par des procédés et/ou traitements spéciaux, par exemple du type ajout de produits enrobants ou gélifiants qui vont limiter et/ou contrôler l'émission des molécules odorantes de l'huile.

Si l'air qui circule à travers le support olfactif est humide, le temps pendant lequel l'odeur est extraite sera plus long. Au contraire, si l'air est sec, on peut imaginer un dispositif permettant de réimprégner le support in ou ex situ ou encore un dispositif à recharge.

Ainsi, si l'on immerge dans un récipient contenant de l'eau, de l'alumine activée imprégnée d'une huile essentielle, on remarque que l'alumine a plus d'affinité pour l'eau qu'elle n'en a pour l'huile essentielle, cette dernière se trouve éjectée (relarguée) et va venir flotter à la surface de l'eau. Cette huile essentielle, surnageant, est analysée par chromatographie en phase gazeuse, couplée avec un spectromètre de masse ; dans le cas de l'alumine activée, les deux spectres de la phase initiale et de la phase désorbée sont sensiblement identiques. On peut donc dire que l'alumine activée se caractérise par sa grande neutralité vis à vis des huiles essentielles, ce qui n'est pas le cas de corps poreux comme par exemple l'atapulgite (à base de magnésium) ou de la sépiolithe (silicate de magnésie) ou tout corps poreux équivalents.

La présente invention concerne aussi la composition contenant une alumine activée imprégnée d'au moins une huile essentielle et/ou de synthèse en vue de son utilisation comme un support olfactif ayant le rôle d'agent odorant et désodorant.

Pour préciser davantage les spécificités de l'alumine activée, on ajoutera que de préférence la porosité de ce support d'alumine activée (sous quelques formes qu'il soit) est constituée de pores de quelques centaine d'A° de diamètre moyen.

Cette porosité est bi-modale, c'est à dire qu'elle est constituée de « rues » qui s'ouvrent dans des « avenues » :
1- de micropores de diamètre moyen compris entre 10 et 90A° (appelons-les « les rues ») lesquels confèrent au support d'alumine sa surface spécifique supérieure notamment à 300 m2 au gramme. Le pourcentage approximatif des micropores est compris entre 60 et 80 %, plus ou moins suivant le type d'alumine choisie.
2- De macropores de diamètre moyen compris autour de 1000A° (appelons-les « les avenues ») lesquels confèrent au support d'alumine leur macroporosité (environ 60 cm3 pour 100 grammes). Le pourcentage approximatif des macropores est d'environ 15%, plus ou moins suivant le type d'alumine choisie.
3- Sur la base de ces caractéristiques, on peut incorporer jusqu'à environ 60 grammes d'huiles essentielles naturelles et/ou de synthèse pour 100 grammes d'alumine activée telle quelle, c'est à dire environ 37,5 % du poids. Dans d'autres cas ce pourcentage peut aller jusqu'à 45%.

Tous les pores communiquent entres eux car ils sont ouverts à leurs deux extrémités ; ce réseau de micropores ou « rues » qui se jettent dans les macropores ou « avenues » peut être rempli par des solutions parfumantes de synthèses et/ou par des huiles essentielles.

EP-A-1285669, FR-A-2816521, XP 002246266 (WAI DERWENT, week 198218) et US-A-5637401 ne suggèrent pas de porosité bimodale dans leurs procédés de déodorisation.

Le support d'alumine activée est très solide et stable dans le temps, c'est à dire qu'il a une très grande résistance à l'écrasement et à l'attrition.
En effet, l'imprégnation d'une solution parfumante de synthèse et/ou d'huiles essentielles peut détériorer le support olfactif eu égard aux propriétés « corrosives » des solutions imprégnées.

Par ailleurs, le support d'alumine est un support extrêmement inerte qui ne modifie pas la nature chimique des bases parfumantes de synthèses et/ou d'huiles essentielles.
En effet, on pourra remplir la porosité de ces perles d'alumines activées par des bases parfumantes de synthèses et/ou d'huiles essentielles et le passage d'un flux d'air à travers un lit de ces perles poreuses créé par différence de températures ou convection naturelle, voire à l'aide d'une ventilation contrôlée va permettre l'évaporation graduelle des bases parfumantes et ainsi réodoriser l'air ambiant en toute maîtrise.

US-A-5 573 984 concerne une déodorisation sur du verre éventuellement à caractère bimodal mais avec des particularités dimensionnelles trés éloignées des notres du fait de la nature du verre.

Si l'on procède à une analyse par chromatographie en phase gazeuse couplée à un spectromètre de masse, on retrouve toujours le même spectre moléculaire que ce soit dans le liquide de départ ou dans la phase gazeuse évaporée comme décrit ci-dessous.

Il n'existe, en effet, aucune interaction physico-chimique entre le support qui est constitué d'aluminé activée sous forme de perles poreuses par exemple et les bases parfumantes de synthèses et/ou d'huiles essentielles que l'on y incorpore.

### II. Conditionnement du support d'alumine activée :

Cette alumine prend place dans une enceinte appelée encore ici « dispositif ».

Comme nous l'avons vu, nous pouvons dire que l'alumine activée imprégnée est un support olfactif qui offre une grande rémanence(persistance de l'odeur) et qui restitue le plus justement tout le spectre moléculaire initial.

Une fois imprégné, le support d'alumine activée doit être le plus souvent conditionné dans une enveloppe inviolable dite transpirante, par exemple : en non-tissé, carton perforé, plastiques à mailles, chanvre, tissus, etc. Un conditionnement qui laisse autant que possible passer le fluide de gaz, d'air, chaud ou froid sur le support d'alumine activée imprégné.

Pour une utilisation du support olfactif d'alumine en vrac, un emballage hermétique est indispensable avant mise en place du support.

Conditionné en fonction d'un modèle d'application déterminé, le support olfactif d'alumine activée est intégré à un dispositif ou non. Le dispositif peut ensuite « se fixer» sur n'importe quelle embouchure au passage d'un fluide d'air ou de gaz, chaud ou froid, comme par exemple un filtre autonome.
Le conditionnement du support olfactif détermine un mode de diffuseur :
- de type cassette ou recharge dans un boîtier spécialement conçu et pouvant être placé face à un système de ventilation intégré,
- de type accroche multiple, filtre autonome à suspendre à proximité de n'importe quel système de ventilation existant (d'un aspirateur, ordinateur, climatiseur, ventilateur, aération, etc),
- de type vrac dans un réceptacle de diffuseur d'odeurs ou tout contenant au contact de l'air ambiant et ventilé par convection naturelle.

### III. L'alumine activée imprégnée associée à d'autres supports imprégnés :

La composition du support olfactif à base d'alumine est constituée à 100% d'alumine activée imprégnée : cette composition est adéquate pour des dispositifs d'odorisation longue durée. Mais le support peut contenir, outre l'alumine, un autre type de support minéral (autre que l'alumine) ou végétal, par exemple de 50 à 100% d'alumine (en poids) et de 0 à 50% de support minéral ou végétal.

Par exemple, la composition du support olfactif est constituée à 50%(en poids) d'alumine activée imprégnée et à 50% (en poids) de pierre ponce imprégnée : cette composition est adéquate pour une odorisation rapide mais de courte durée.

On peut mélanger à l'alumine activée imprégnée, un produit à base de support minéral ou végétal afin de créer un effet olfactif dit « choc ». En effet, l'alumine activée absorbe la solution d'imprégnation jusque dans son coeur, alors que tout autre support, par exemple la pierre ponce absorbe la solution d'imprégnation superficiellement en surface. Ainsi au contact d'un fluide, c'est le support pierre ponce qui va libérer en premier les arômes, d'où l'effet « choc », alors que le support d'alumine imprégné, libérera ses arômes dans la durée. La pierre ponce ou tout autre support, aura donc libéré la totalité de ses arômes, alors que le support d'alumine continuera à libérer les siens dans la durée.
Comme exemple de minéraux, on citera la pierre ponce, le sable, les argiles, les silices, etc.
Comme exemple de végétaux, on citera la rafle de maïs, le blé concassé, le bois, etc.

Ce support minéral ou végétal contient donc lui aussi un agent d'odorification ou de déodorification qui peut être un complexe parfumant d'huiles essentielles et/ou d'huiles de synthèse ou tout autre agent adéquat.

### IV. A propos des solutions parfumées servant à l'imprégnation de l'alumine activée :

On peut imprégner sur le support d'alumine des huiles (solutions parfumées) odorantes de synthèses et/ou des huiles essentielles de première pression à froid.
Certaines huiles vont mieux tolérer l'imprégnation, du fait de leurs propriétés physico-chimiques. On procèdera alors à une imprégnation par paliers ou monocouches en respectant un ordre d'imprégnation suivant les solutions à imprégner.

Ci-dessous quelques exemples de formulations à base d'huiles essentielles :
- Pour 100 ml de solution parfumée à base d'huiles essentielles :
   - 20 ml de romarin + 20 ml de camphre + 30 ml de lavande + 30 ml d'orange,
   - 50 ml d'eucalyptus + 20 ml de menthe poivrée + 30 ml de camphre,
   - 30 ml de pamplemousse, 10 ml de ravensara, 20 ml de citron, 40 ml d'orange.

On peut imprégner des huiles de synthèse c'est-à-dire des solutions qui restituent toutes sortes d'arômes, par exemple des odeurs suaves, type : chocolat, miel, cannelle, etc. Les huiles de synthèse ont une tendance à « sucrer » les fragrances qui contiennent des huiles essentielles pures.
Outre les qualités olfactives des huiles essentielles, la plupart d'entre elles possèdent des propriétés bactéricides et/ou anti virales d'où leur caractère purifiant et dépolluant.

Si l'huile de synthèse et/ou essentielle constitue la base de la solution parfumée à incorporer dans l'alumine activée, on peut ajouter à cette solution certains principes actifs afin de répondre à une application déterminée, par exemple, sans que cette liste soit limitative :
- fongicides et/ou anti-acariens, par l'adjonction de pyréthrine ou composé équivalent et/ou d'huiles essentielles spécifiques dans la formulation de la solution.
- actifs chimiques divers, non toxiques, de types dépolluants.
- phéromones connues à ce jour, complexe phéromonale.

### V. Conclusions:

L'utilisation de l'alumine activée comme support olfactif de base répond à :
- une plus grande rémanence olfactive (durée de l'odeur),
- une neutralité olfactive du support vierge,
- un conditionnement du support à la carte,
- une adaptation du support à tous types de fluides,
- une flexibilité en terme de dispositifs d'odorisation,
- une souplesse en matière de fabrication (écologique),
- une hygiène du support conditionné ou non,
- un intérêt économique dans différentes applications,
- une esthétique du support par les couleurs et les formes différentes,
- une restitution parfaite de la solution parfumée imprégnée.

## Revendications

1. Procédé d'odorisation ou de désodorisation d'un gaz consistant à faire circuler le dit gaz, chaud ou froid, à travers une enceinte renfermant un support contenant de l'alumine activée, la dite alumine étant imprégnée d'au moins une huile essentielle et/ou de synthèse et procédé dans lequel la dite alumine activée comporte une porosité bi-modale constituée de micropores de diamètre moyen compris entre 10 et 90 A° et de macropores de diamètre moyen 1000A°.

2. Procédé selon la revendication 1 dans lequel l'alumine activée possède une surface spécifique comprise entre 200 et 500 m2 par gramme et un volume poreux compris entre 20 et 70 cm3/g.

3. Procédé selon l'une des revendications 1 et 2 dans lequel le dit support renferme, outre de l'alumine, un produit à base de support minéral ou végétal, la teneur en poids d'alumine étant comprise entre 50 et 100%, la teneur en poids en support minéral ou végétal étant comprise entre 0 et 50%.

4. Procédé selon la revendication 3 dans lequel le support minéral est la pierre ponce, le sable et les argiles.

5. Procédé selon la revendication 3 dans lequel le support végétal est la rafle de maïs, le blé concassé et le bois.

6. Procédé selon l'une des revendications 1 à 5 dans lequel on ajoute au dit support d'alumine activée au moins un produit bactéricide ou fongicide, antiacarien, ou à base de phéromone.

7. Procédé selon l'une des revendications 1 à 6 dans lequel on ajoute au support d'alumine activée un agent enrobant ou gélifiant pour agir sur la rhéologie du système support/huile.

8. **Dispositif ou enceinte à travers lequel ou laquelle on fait circuler un gaz ou de l'air** (chaud ou froid) à odoriser ou désodoriser, ayant une forme de type cassette, réceptacle ou recharge et renfermant un support défini comme aux revendication 1 à 7

9. Dispositif ou enceinte selon la revendication 8 dans lequel plus particulièrement le dit support est contenu dans un conditionnement de type cassette ou recharge placé dans un boîtier, de type accroche multiple, de type « en vrac » disposé dans un réceptacle. 10 Composition à base d'un support contenant (a) de l'alumine activée imprégnée d'au moins une huile ou d'une solution d'une huile essentielle ou de synthèse, cette alumine activée étant capable de diffuser progressivement l'huile qu'elle contient et (b) un support minéral ou végétal, autre que l'alumine, contenant un agent d'odorification ou désodorification, ce support minéral ou végétal diffusant rapidement l'agent qu'il contient, la dite alumine activée étant **caractérisée en ce qu'**elle comporte une porosité bi-modale constituée de micropores de diamètre moyen compris entre 10 et 90 A° et de macropores de diamètre moyen 1000A°.

## Claims

1. Method and device for gas odorization or deodorization consisting of causing said gas, warm or cold, to move through a vessel (enclosure) comprising a support which contains activated alumina, said alumina being impregnated with at least one essential oil and / or one synthesis oil and method wherein said activated alumina comprises a bi-modal porosity consisting of micropores whose the average diameter is comprised between 10 and 90 angstroms and of macropores whose the average diameter is 1000 angstroms.

2. Method or process according to claim 1 wherein the activated alumina has a specific surface between 200 and 500 m2/gram and a pore volume between 20 and 70cm3/gram .

3. Method according to one of claims 1 and 2 wherein said support contains, besides alumina, a substance containing a mineral support or a vegetable support, the weight amount of alumina being comprised between 50 and 100%, the weight amount of the mineral support or of the vegetable support being comprised between 0 and 50%.

4. Method according to claim 3 in which the mineral support is pumice-stone, sand and clays.

5. Method according to claim 3 in which the vegetable support is col of maise, grinded corn and wood.

6. Method according to one of claims 1 to 5 in which at least one bactericidal or fungicidal substance, acaricidal substance, or with pheromone, is added to the said activated alumina support.

7. Method according to one of claims 1 to 6 in which a coating or gelling substance is added to the activated alumina support in order to act on the rheology of the support/Oil system.

8. Device or enclosure through which a gas or air, warm or cold, that needs to be subjected to odorization or to deodorization, is moved, said device or enclosure having a cassette form, a receptacle form, a replacement form or a form of the like type and containing a support according to one of claims 1 to 7.

9. Device or enclosure according to claim 8 in which more particularly the said support is contained in a conditioning device of the cassette or of the like type device or of the replacement type, that is put in a box of multiple hanger type or of the in bulk type that is put in a receptacle.

10. A support-based composition which contains:
(a)activated alumina impregnated at least with an oil or a solution of an essential oil or of a synthesis oil, this activated alumina being able to progressively diffuse the oil that it contains and
(b)a mineral support or a vegetable support, other than alumina, containing a scenting agent or a deodorization agent, this mineral support or this vegetable support rapidly diffusing the agent that it contains, the said activated alumina being **characterized by** comprising a bi-modal porosity consisting of micropores whose the average diameter is between 10 and 90 angströms and of macropores whose the average diameter is 1000 anströms.

## Patentansprüche

1. Verfahren zum Wohlriechendmachen oder Geruchlos machen von einem gas, bestehend aus dem Zirkulierenlassen des warmen oder kalten Gas, durch einen Raum der einen Träger fasst (enthält), der eine aktivierte Tonerde zumindest mit mindestens einem ätherischen Öl und / oder einem synthetischen Öl imprägniert ist und Verfahren wobei die besagte aktivierte Tonerde eine Porendoppelstruktur besitzt, die Mikroporen mit einem mittleren Durchmesser zwischen 10 und 90 angströms und Makroporen mit einem mittleren Durchmesser von 1000 angströms beträgt.

2. Verfahren nach Anspruch 1 wobei die aktivierte Tonerde eine spezifische Oberfläche zwischen 200 und 500 m2/gram und ein Porenvolumen zwischen 20 und 70 cm3/g besitzt.

3. Verfahren nach einem der Ansprüche 1 und 2, bei welchem der besagte Träger, außerdem Tonerde, ein Produkt das einen Mineralträger oder einen Pflanzenträger enthält, im Bereich 50-100 Gew.-% Tonerde, 0-50 Gew.-% Mineralträger oder Pflanzenträger.

4. Verfahren nach Anspruch 3 bei welchem der Mineralträger Bimsstein, Sand und Tone ist.

5. Verfahren nach Anspruch 3 bei welchem der Pflanzenträger Maistraubenstrunk, geschrottet Korn und Holz ist.

6. Verfahren nach einer der Ansprüche 1 bis 5 wobei man der besagten aktivierte Tonerde mindestens einer bakteridizen oder fungiziden Stoff oder gegen Milben Stoff oder enthaltende Pheromone Stoff hinzufügt.

7. Verfahren nach einer der Ansprüche 1 bis 6 wobei man der aktivierten Tonerde einen einkapselnden oder gelierende Stoff hinzufügt, um zu über die Rheologie des Träger / Öl System zu bewirken.

8. Vorrichtung oder Raum wo man ein Gas oder Luft (warm oder kalt) zirkulieren lässt damit man dieses Gas oder diese Luft wohlriechend macht oder Geruchlos macht, wobei die Vorrichtung vom Typ Kassette, Sammelbecken, Nachfüllung ist, und wobei die besagte Vorrichtung einen Träger, der gemäss einem der Ansprüche 1 bis 7 definiert ist, enthält.

9. Vorrichtung oder Raum nach dem Anspruch 8 wobei besonders der besagte Träger in einer Anlage vom Typ Kassette oder Nachfüllung erhalten ist, der in einem Fächerkasten liegt, vom Typ Anhänger, vom Typ unverpackt der in einem Sammelbecken gestellt ist.

10. Verbindung mit einem Träger der enthält:
(a) aktivierte Tonerde die mindestens mit einem Öl oder mit einer Lösung eines ätherischen oder synthetischen Öl imprägniert ist, wobei diese aktivierte Tonerde, die das Öl enthält, fähig ist das Öl allmähig ausbreiten,
(b) einen Mineralträger oder einen Pflanzenträger, außerdem Tonerde, der einen Wirkstoff zum Wohlriechendmachen oder Geruchlosmachen enthält, wobei dieser Mineral oder Pflanzenträger Wirkstoff schnell verbreiten, den er enthält, **dadurch gekennzeichnet dass** die aktivierte Tonerde eine Porendoppelstruktur mit Mikroporen mit einem mittleren Durchmesser Durchmesser zwischen 10 und 90 Angströms und Makroporen mit einem Mittleren Durchmesser von 1000 Angströms beträgt.
